# EUROPEAN PATENT SPECIFICATION

(11) **EP 2 572 189 B1**
(45) Date of publication and mention of the grant of the patent: **19.07.2017**
(21) Application number: 11721299.3
(22) Date of filing: 20.05.2011
(51) Int. Cl.: G01N 27/447, G01N 33/53, G01N 33/68, G01N 1/04

(54) **WESTERN BLOT ANALYTICAL TECHNIQUE**
WESTERN-BLOT-ANALYSEVERFAHREN
TECHNIQUE ANALYTIQUE DE TRANSFERT WESTERN

(30) Priority: 05.01.2011 GB 201100092; 21.05.2010 GB 201008517
(43) Date of publication of application: 27.03.2013
(73) Proprietor: Agilent Technologies, Inc., Santa Clara CA 95051 (US)
(72) Inventor: MACNAMARA, Kenneth G, Edinburgh EH10 5NH (GB); POLWART, Stuart, Banknock FK4 IUE (GB)
(74) Representative: Dilg, Haeusler, Schindelmann Patentanwaltsgesellschaft mbH
(86) International application number: PCT/EP2011/058306
(87) International publication number: WO 2011/144755

(56) References cited:
- US-A- 5 238 651
- US-A1- 2005 277 185
- US-B1- 6 680 208
- PAL J K ET AL: "Staining of proteins on SDS polyacrylamide gels and on nitrocellulose membranes by Alta, a colour used as a cosmetic", JOURNAL OF BIOCHEMICAL AND BIOPHYSICAL METHODS, AMSTERDAM, NL, vol. 61, no. 3, 30 November 2004 (2004-11-30), pages 339-347, XP004663695, ISSN: 0165-022X, DOI: 10.1016/J.JBBM.2004.06.008
- ROBERT J. MEIER ET AL: "SDS-PAGE of Proteins Using a Chameleon-Type of Fluorescent Prestain", ANALYTICAL CHEMISTRY, vol. 80, no. 16, 15 August 2008 (2008-08-15), pages 6274-6279, XP55009416, ISSN: 0003-2700, DOI: 10.1021/ac800581v
- ALDRIDGE G M ET AL: "The use of total protein stains as loading controls: An alternative to high-abundance single-protein controls in semi-quantitative immunoblotting", JOURNAL OF NEUROSCIENCE METHODS, ELSEVIER SCIENCE PUBLISHER B.V., AMSTERDAM, NL, vol. 172, no. 2, 30 July 2008 (2008-07-30) , pages 250-254, XP026863323, ISSN: 0165-0270 [retrieved on 2008-07-30]

## Description

### Field of the invention

This patent application relates to an improved method of performing the western blot analytical technique.

### Technological Background

Western blotting is a labour intensive laboratory analysis method that is widely used in the life sciences to determine whether a target protein is present in a complex sample and to determine the relative quantity of the target protein. The phrase "target protein" is used to refer to a protein that a user of an analysis method wishes to identify within a complex sample. The relative quantity of a protein is used to measure changes in protein expression (i.e. up regulation and down regulation).

Determining whether a particular protein is present is achieved by connecting two variables: the molecular weight of the protein and its immune identity (with the assumption that it is unlikely that these two very different aspects of a protein coexist by chance). Determining the relative quantity of a particular protein is achieved by comparing the quantity of the target protein in the complex sample to either the total protein content or the quantity of a common "housekeeping" protein. The term "housekeeping" protein is used to refer to a common protein involved with basic functioning of a cell, for example beta-actin or tubulin. A second function of quantifying the total protein or a "housekeeping" protein in western blot analysis is that they can be used as a loading control. The quantity of the total protein or a "housekeeping" protein present can be used to identify inaccuracies in lane loading and sample preparation. Target protein values are then normalised using these differences so that these inaccuracies are taken into account.

The standard western blot method separates the proteins in a complex sample using electrophoresis (e.g. sodium dodecyl sulfate polyacrylamide gel electrophoresis, SDS-PAGE), then electro-transfers the separated proteins to a solid membrane (commonly made from nitrocellulose or polyvinylidene fluoride, PVDF) such that the proteins retain the same separation pattern. This membrane is then incubated in diluted protein solutions, e.g. non-fat dry milk or bovine serum albumin (BSA), to block the non-specific binding sites, then incubated with a primary antibody that is specific for the target protein, washed and incubated with a secondary antibody that allows for detection of the target protein. This is known as immunodetection. Once a user has determined whether a target protein is present in the sample, the primary and secondary antibodies are stripped from the membrane, and the membrane is incubated with an alternative primary antibody that is specific for another protein that may be used as a loading control, washed and incubated with a secondary antibody that allows for detection of the loading control.

The whole method typically takes 5 to 8 hours to complete, although some users prefer to leave some steps within the overall method to run overnight.

Problems with the standard western blot method include the fact that following electrophoresis the separated protein sample cannot normally be visualised and so a user cannot readily analyse the results of the protein separation. The separated protein sample could be stained after separation so that a user can visualise the separated proteins, however, this is both labour intensive and time consuming.

For example, Aldridge et al., Journal of Neuroscience Methods 172 (2008) 250-254 describes an immunoblotting method wherein a protein sample, whose protein concentration was determined using a bicinchoninic acid (BCA) protein assay test, was denaturated, subjected to PAGE and transferred to a nitrocellulose membrane. The blots on the nitrocellulose membrane were stained with SYPRO^{®} Ruby or - after blocking and antibody incubations - stained with Amido Black. These two protein stains were found to be acceptable alternatives to high-abundance single-protein controls, such as housekeeping proteins.

One further method commonly used for detection of proteins post separation and prior to immunodetection is exposing the separation medium to Ponceau S stain. This methodology requires labour intensive staining and destaining steps before the proteins are rendered visible. The proteins revealed using this technique are often faint and lacking the contrast required to capture digital images. Also, as current methods of immunodetection usually detect the target protein using chemiluminescence and photographic film the image of the total protein from Poceau S staining will not be directly comparable to the image of the target protein.

Another methodology commonly used to compare the amount of total protein to target protein using two identical electrophoresis protein separations. One separation medium is stained for detection of total protein and the other transferred to a solid membrane for immunodetection of the target protein. This method is an unattractive option in many situations especially with valuable samples or where there is only a small sample volume. Also, as the majority of people load samples into electrophoresis apparatus manual comparison of protein content between different electrophoresis separations can lead to the incorrect interpretation of the result due to inexact loading of the sample.

This demonstrates that the additional process steps required to determine protein expression incur additional costs in the form of reagents and time. In addition, if a standard western blot method is used, a user cannot readily compare the results from the same separated protein sample before and after immunodetection.

Another problem with the standard western blot method is that it comprises multiple steps involving several different apparatuses thereby creating barriers to integration, including a separate device being needed to image the final sample after it has been probed. Additionally, many of these multiple steps involve wet chemistry which can lead to a lack of reproducibility. For example, the most widely used method of detecting a target protein is by chemiluminescence involving exposing a photographic film to the western blot. Using photographic film requires the blot to be transferred to a dark room, which is often some distance from the laboratory, in order to position the photographic film in contact with the blot. The photographic film processing units required to develop the exposed film are expensive and often difficult to maintain. Another difficulty of using photographic film is that obtaining an unsaturated image of the target protein suitable for quantitative analysis requires time consuming trial and error.

Pal et al., J. Biochem. Biophys. Methods 61 (2004) 339-347 evaluates the suitability of a specific dye, Alta, commonly used as a cosmetic, for staining proteins on SDS polyacrylamide gels and on nitrocellulose membranes. The authors conclude that Alta represents a highly inexpensive stain which can be used as an alternative to Coomassie Brilliant Blue for staining proteins in protein electrophoresis and Western Blot analysis. Overall, the standard western blot method is labour intensive, time consuming and uses a significant quantity of reagents.

### Summary of the Invention

Hence, there may be a need for a Western blot analytical technique being sufficiently rapid and efficient.

Embodiments of the present invention provide an improved method of performing the western blot analytical technique that overcomes many of the above problems by providing a rapid, efficient and convenient alternative. The invention in its general sense is a method of performing the western blot analytical technique, wherein the method comprises carrying out the following steps in the following order:
a). pre-staining the proteins within a sample;
b). separating the proteins using gel electrophoresis;
c). analysing the separated proteins to determine the total protein load and/or at least one housekeeping protein load;
d). transferring the proteins onto at least one membrane;
e). probing the separated proteins to detect a target protein; and
f). analysing the probed proteins to determine the target protein's molecular weight and load,
wherein the total protein load and/or housekeeping protein load are compared to the target protein load to find the ratio of total protein and/or housekeeping protein load to target protein load, and wherein the total protein load and/or housekeeping protein load are used as a loading control and the ratio of the total protein and/or housekeeping protein load to target protein load is normalised using these values.

The present inventors have developed a technique which overcomes at least a part of the following short comings: A scientist carrying out a typical Western blot may look to compare samples within an experiment and with other experiments, for example, to compare the level of expression of the target protein. Often this is done by eye, determining whether the target protein is brighter than another. This method is very prone to error in loading and initial sample preparation. More rigorously, and usually expected for publication of results, the brightness of a target protein needs to be compared to a loading control protein. To do this comparison, the membrane needs to be stripped and re-probed for the loading control protein which adds time and further complexity to the experiment.

As compared to such conventional approaches, an exemplary embodiment of the invention provides the advantage that all proteins in the sample are pre-stained (step a). This enables the instrument to measure the amount of total protein load or a loading control protein following the electrophoresis (steps b and c). Once the proteins have been transferred to the membrane and the Western blot carried out (steps d and e), the membrane can be re-analysed in the same instrument to determine the target protein's molecular weight and/or load (step f) relative to the total load or loading control (measured in step c). It is a significant advantage over present methods in that it does not require stripping and re-probing of the membrane to measure the loading control and because the analysis is carried out in the same instrument making use of alignment features the images following electrophoresis and following the Western blot can be directly compared.

The scientist, by use of such embodiments of the invention, may also analyse the membrane following transfer and use the transferred results as a quality control step to ensure good transfer and as a loading control. In standard methods the scientist would have to stain with Ponceau S following transfer as a QC test and then de-stain before probing. Ponceau S images are not easily comparable to images following Western blot due to the difficulties of imaging Ponceau S and the different method of imaging following Western blot.

A further advantage of an embodiment is that it is not necessary to run a specific Western blot molecular weight standard. Due to the alignment of the original image following electrophoresis to the Western blot membrane, the molecular weight standard from electrophoresis provides the calibration for molecular weight.

The method of an embodiment of the present invention allows a user to check that step b) and d) have been successful before investing time and effort in probing the separated proteins. Additionally, using the results of the analysis of the separated proteins to determine the total protein load negates the need to re-probe for a loading control after step e).

The data generated from using this method is then used to perform semi-quantitative analysis of the target protein across different lanes and different samples. The total protein load and/or housekeeping protein load are then compared to the target protein load to find the ratio of total protein and/or housekeeping protein to target protein. The total protein load and/or housekeeping protein load are also used as loading controls. This allows for accurate lane-to-lane comparison of target protein levels.

The preferred embodiment of calculating the ratio of total, or housekeeping, protein to target protein uses an appropriate imaging system capable of detecting and recording the fluorescence produced by a fluorophore excited at a specific wavelength, for example the LAB901 TapeStation. The data produces a graph of intensity to distance along an electrophoresis gel. The area under this curve for total protein, or in the case of a housekeeping or target protein a single peak, is taken to be the "load". The ratio of total or housekeeping protein load to the target protein load can then be calculated. In another embodiment, capillary electrophoresis rather than gel electrophoresis might be used to separate a mixture of proteins in a sample. In such circumstances the distance axis is replaced with a time axis as the measurement of intensity of fluorescence would be measured at a fixed point as the biomolecules move through the capillary. Therefore, the intensity is plotted against the time said fluorescence is detected at a fixed point from the start of protein separation.

There are several methods which can be employed to determine the concentration of target protein present in the sample. One such method would be to produce a calibration curve using known concentrations of a protein. For example, a protein at known concentrations can be used in a Western blot analysis assay and the load values of this analysis used to create a calibration curve. The target protein load from a Western blot analysis can then be read from this calibration curve to give the concentration of the target protein within a given sample. The protein used to generate the calibration curve would, preferably, be known concentrations of the target protein, however, in many circumstances this may not be possible and a different protein could equally be used to generate such a calibration curve. For example, bovine serum albumin (BSA) and bovine gamma globulin (BGG) are commonly used to generate calibration curves in protein quantitation assays. Calculating the target protein load may be performed with the aid of appropriate software and a programmable computer. The accuracy of this calculation may be increased by using a dilution series of the protein sample. Another method may involve comparing the target protein load and the known load of in lane markers.

The total protein load and at least one housekeeping protein load can both be measured to be used as loading controls. The inclusions of two loading control calculations may improve the overall accuracy of the adjustments made to the measurements of target protein.

Various compounds may be used to pre-stain the proteins, including fluorophores such as pyrillium based dyes available from Active Motif, the Alexa Fluor dyes available from Molecular Probes, the Dylight dyes available from Thermo Fisher Scientific, the Atto dyes available from Atto Tec and the CyDyes available from GE Healthcare. A single fluorophore or more than one fluorophore may be used to pre-stain the proteins.

Upon completion of gel electrophoresis the separated proteins are located within the matrix of the gel. Gels for use in gel electrophoresis may be made by casting into a mould at point of use, which can enable easy access to the separated components within the gel after use. However, pre-packaged gels (in which the gel is packaged within a container), such as the Lab901 ScreenTape^{®} range have become more popular for use in gel electrophoresis, especially for protein separation. This is because pre-packaged gels are more convenient to use, provide more reproducible results and are safer to use and dispose. Therefore, when pre-packaged gels are used in gel electrophoresis as an upstream preparative technique prior to a downstream analytical technique, the separated components may need to be accessed and extracted from within the pre-packaged gels. Therefore, the method of an embodiment of the present invention may comprise the additional step of accessing the gel from within a container following gel electrophoresis but prior to analysing and/or probing the separated proteins. Additionally, a programmable computer could be used to control the voltage and time used during gel electrophoresis.

The step of separating the proteins using gel electrophoresis will typically enable a one dimensional separation of the proteins, however, two dimensional separation of the proteins is also possible. One dimensional separation is useful for separating proteins according to their molecular weight whilst two dimensional separation is useful for separating proteins according to their weight and their isoelectric point.

Analysing the separated proteins to determine the total protein load commonly involves capturing an image of the pre-stained separated proteins within the gel.

The separated proteins will typically be transferred from the gel to at least one membrane to assist in the probing of the separated proteins. Traditionally, the transfer process involves movement of protein samples on a single large electrophoresis gel to a single sheet of membrane. If the protein samples need to be probed independently the membrane has to be cut into individual sections containing the protein samples. Although this method is suitable for use in an embodiment of the present invention, it is a time consuming and imprecise method of independently probing multiple samples. A more advantageous approach would be to use the Lab901's ScreenTape^{®} technology, which provides a means of conducting 16 protein separations simultaneously on 16 separate pre-packaged gels (as described in WO 06/085071).

If pre-packaged gels are used to separate the protein sample then some form of gel extraction apparatus may be used to ensure the molecular separation gel is extracted from a container by moving the gel relative to the container. Extraction of the gel from the container may comprise sucking, blowing, pushing, pulling, or flushing out the gel. However, the most preferable means of extraction is to move the gel relative to the container by pushing the gel. Pushing the gel may be achieved using at least one gel pushing element acting on the container, for example a plurality of gel pushing elements may act on a plurality of sub-containers within the container. A plurality of gel pushing elements may form a comb and the said pushing elements may be guided into location. Preferably the part of each gel pushing element that contacts the surface of the gel substantially corresponds in shape and dimensions to the cross-sectional area of the interface between the pushing element and the gel. This helps ensure that the whole gel is displaced from the container whilst maintaining the aspect ratio of the gel. However, the gel pushing elements may be resiliently deformable to allow tolerance for small changes in the interior of the sub-containers shape and dimensions, and the alignment of the gel pushing elements with the container. One means of providing resiliently deformable gel pushing elements, is to split the end of each gel pushing element into separate lobes separated by a slit. Another means of providing resiliently deformable gel pushing elements could involve using a coating of an elastomer or wax at the tips of the pushing elements.

An embodiment of the present invention may also include the steps of physically manipulating the gel container to aid extraction of the gel. For example, rolling the container, gently applying pressure or massaging the container to minimise surface tension between the gel and the container surfaces. This physical manipulation may be done manually of by a mechanical process.

The transfer process of step d uses an electric field to transfer the proteins from the gel to at least one membrane. Ensuring full transfer of proteins from a gel to a membrane can be critical for the success of a Western blot. Therefore, a programmable computer can be used to control the voltage and time used during transfer of the separated proteins onto the at least one membrane.

Transferring the separated proteins may enable a user to divide a larger sample of the separated proteins into smaller sub-samples of the separated proteins, which allows different probes to be used on each sub-sample. Alternatively, a single probe at different concentrations can be used on each sub-sample to optimise the probing protocol. In another embodiment multiple samples of separated proteins may be transferred enabling a user to probe multiple samples of separated proteins simultaneously.

Multiple membranes may be used for multiple sub-samples or multiple samples of separated proteins. Alternatively, a single membrane may be used for multiple samples or multiple sub-samples.

Using a single membrane separated into sections allows smaller volumes of probe solutions to be used, further optimising the probing of the separated proteins. In embodiments in which the protein separation is carried out using Lab901's ScreenTape technology up to 16 samples of separated protein may be transferred onto a single membrane separated into 16 sections.

Suitable membranes for use in the present method include those comprising PVDF or nitrocellulose. Preferably the membrane is hydrophilic or has been treated to be hydrophilic since this can improve contact between the separated protein sample and the surface of the membrane.

In another embodiment the transfer to the membrane is performed using optimised conditions to prevent smaller proteins migrating through the membrane faster than the larger proteins, a phenomenon know as "blow through". Blow through leads to differences in protein distribution between the surface in contact with the electrode and the surface in contact with the gel. To address this problem the method described here may contain the added steps of carrying out the transfer in optimised buffers to prevent blow through. The optimisation of the buffers may include varying the transfer buffer pH, buffer type, use of discontinuous buffers, i.e. different buffers in the anode and cathode blotting paper and different again in the membrane, or by varying the voltage per cm across the gel. Varying the voltage per cm across the gel provides a less strong electric field at the bottom of the gel, where the small proteins migrate to after electrophoresis, and a stronger electric field at the top of the gel, where the larger protein migrate to after electrophoresis.

In another embodiment the blotting paper used in the process is of a certain quality and smoothness. A critical factor in ensuring good protein transfer can be ensuring good contact between the blotting paper and the membrane. Poor contact can result in uneven transfer and a "blotchy" appearance to the final image of the protein which inhibits good analysis.

As previously state, traditional Western blot methods transfer proteins from a single electrophoresis gel to a single membrane. If the protein samples transferred to a membrane need to be probed independently, then the membrane must be cut into individual sections. In embodiments where the pre-packaged Lab901 ScreenTape^{®} technology is used and the gel fragments extracted, as described above, onto a single membrane, then different samples, or differential treatment of identical samples in different lanes, can be performed individually using membranes treated to prevent fluid transfer between lanes. Sections of membranes, such as PVDF, can be treated using heat or ultrasonic welding to produce the individual protein transfer zones. The treatment procedure solidifies the membrane around these sections producing liquid tight barriers. These liquid tight barriers prevent the flow (wicking) of fluid from one lane to another such that only untreated areas allow fluid to penetrate the membrane.

Therefore, individual protein transfer zones are created in which proteins can be transferred and subsequently treated independently.

An additional step post-transfer and prior to target protein detection could be capturing an image of the electrophoresis gel post transfer as a quality control step for transfer efficiency. Comparison of this image and the image of the gel taken pre-transfer can be used to ensure that complete transfer of the proteins has occurred. Inclusion of this step could save valuable time and resources if protein transfer has been incomplete or unsuccessful. Another quality control step could be to image the total protein transferred to the at least one membrane. Capturing an image of the separated proteins after they have been transferred onto the at least one membrane allows for inspection of the transferred proteins to ensure there has been no warping or distortion of the proteins during the gel extraction and transfer steps.

After verification that the transfer process has occurred successfully the membrane can then be probed to detect the target protein in step e. Traditionally, this process involves a large degree of physical manipulation of the membrane and large volumes of incubation solutions e.g. antibody solutions. The membrane is first blocked to prevent non-specific binding of the primary antibody to the membrane. Primary antibody incubation is then applied to the membrane and incubated on a rocker. Following this incubation the primary antibody solution is removed and washed several times. Secondary antibody is then applied and again incubated with shaking. The secondary antibody is then removed and the membrane washed again. This traditional approach to probing of a membrane is suited to an embodiment of the current invention, however, an incubation device could also be used to reduce the volumes of liquid required and to enable probing of different samples lanes individually.

The incubation device may comprise a mask and at least one membrane, wherein apertures in the mask form isolated sections. The isolated sections are formed by the mask comprising raised barriers, and wherein at least one membrane is located within each of the isolated sections. The incubation device may also include an overflow area formed around the sections such that larger volumes of liquid can be used to cover all of the sections simultaneously. Therefore, liquid may be placed in small volumes into the section to treat the sections of the membrane independently or larger volumes of liquid can be placed over all of the samples filling all sections but maintaining the liquid in a manageable area.

The incubation device may comprise plastic and may incorporate alignment features. Using said alignment features locations on the mask could be mapped back to corresponding alignment features on a container used during gel electrophoresis. These alignment features assist in the comparison of the results of protein separation and the results of incubation.

The incubation device may also include fluid tight deformable gaskets at the interface with the membrane forming a liquid tight seal around the isolated section. Therefore, fluid placed on one lane cannot flow over the membrane to the adjacent lanes due to the fluid tight deformable gasket and cannot flow through the membrane to the adjacent lanes due to the heat treatment. The fluid tight deformable gaskets, which are of the precise dimensions to sit around the individual protein transfer zones, will aid the formation of liquid tight barriers preventing cross contamination between sample lanes. Therefore, individual protein transfer zones can be designed to align precisely to the isolated sections of the incubation device.

Alternatively, the incubation device maybe separated into isolated sections by hydrophobic barriers. These hydrophobic barriers may comprise a glue and/or an ink which is applied by screen printing.

Fluid applied to the sections created by the incubation device could be removed using a means to pull a vacuum incorporated into the incubation device. Vacuum aspiration could be used to pull the fluid through the membrane to a waste container positioned there under.

Detecting the target protein typically involves detection of a luminescent or fluorescent label that is covalently bonded to the target protein during step e) using a one or two step method. In the one step method the label is covalently bonded to at least one antibody capable of binding to the target protein. In the two step method at least one unlabelled antibody (primary antibody) capable of binding to the target protein is incubated with the membrane, then the membrane is washed and incubated with at least one antibody (secondary antibody) which is covalently bonded to the label and is capable of binding to the primary antibody.

One example of a commonly used chemiluminescent immunodetection label is horseradish peroxidase (HRP). This enzyme is conjugated to an antibody and used to convert a non-luminescent substrate into a luminescent product which can be detected using photographic film. As the number of antibodies bound to the membrane increases with increasing quantities of target protein, so too does the amount of luminescent product produced by the HRP enzyme conjugated to those antibodies.

When fluorophores are conjugated to antibodies and used for protein detection the procedure is much the same as previously stated with the exception that a chemiluminescent substrate is not required. Instead the fluorophore is excited at a specific wavelength and the fluorescence produced recorded by an appropriate imaging system.

Analysing the probed proteins to determine the target protein load commonly involves capturing an image of the target protein. Comparing the image of the probed proteins with the image of the separated proteins enables a user to determine the amount of target protein present in a sample. Therefore, the total protein and/or housekeeping protein can be associated with a fluorophore and the target protein can be associated with fluorophore which excites and emits at a different wavelength from the fluorophore associated with the total protein and/or housekeeping protein.

Analysing the separated proteins and analysing the probed proteins may comprise manipulation of the analytical data using software analysis techniques. Preferably, a single analytical device is used to analyse the separated proteins to determine the total protein load and/or housekeeping protein and to analyse the probed proteins to determine the target protein load. For example, the TapeStation^{®} (available from Lab901) may be used in both analytical steps. The TapeStation^{®} can detect multiple wavelengths and so in embodiments in which detection of one fluorophore enables a user to determine the total protein load and detection of another fluorophore enables a user to determine the target protein load, the TapeStation^{®} can be used to measure changes in protein expression. Similarly where detection of one fluorophore enables a user to determine the load of a "housekeeping" protein and detection of another fluorophore enables a user to determine the target protein load, the TapeStation^{®} can be used to detect both the quantity of a "housekeeping protein" and to detect the total protein load enabling semi-quantitative analysis. The calculated quantity will not necessarily be accurate, however, the ratio between the target and total load is precise and changes in expression can be determined and quantified precisely using this ratio.

Alignment features (such as punch holes, reference edges, datums, optical fiducials, formed features or any combination thereof) may be used to assist in mapping the results of the analysis of the separated proteins and the results of the analysis of the probed proteins. The gel container may comprise alignment features which can be used in conjunction with alignment feature of the support to determine the positions of the gel section in relation to their original position within the electrophoresis container. These alignment features depending on the nature of the alignment feature, be automatically processed by, for example, a digital image capture device (such as a CMOS or CCD digital camera) and associated pixel analysis via software installed on an analytical device. Preferably both analytical steps are carried out on the same device, e.g. the TapeStation^{®} as sold by Lab901, as this also assists in the mapping of the results of the analysis of the separated proteins and the results of the analysis of the probed proteins.

By being able to map the target protein back precisely to the original analysis it is possible to identify the target protein peak within the first separation. This peak in the first separation can then be used for accurate quantification without dependency on antibody affinities. Alternatively, if the target protein is a known protein that can be isolated and quantified independently, a dilution series of the sample can be run to provide a calibration curve that an unknown sample can be back-calculated from for accurate quantification.

The device(s) used in the protein separation and protein transfer processes can be controlled using a single computer which can be used to set the voltage and time for both these processes. The time and voltage profile can be selected by a user from one of a set of predetermined programs optimised for various types of protein or one programmed by the user (which they may have optimised to meet their specific requirements).

Another means of assisting in mapping the results of the analysis of the separated proteins and the results of the analysis of the probed proteins is the use of in-lane markers, which may be added during step a). In the context of an embodiment of the present invention, "in-lane markers" refer to proteins that may be added to the sample prior to separation, which proteins will adopt a position near the top or bottom of the gel column following separation. Therefore, the in-lane markers allow a user to identify known protein fragments following separation, and thereby allow the user to predict the size of other protein fragments in the sample by comparison with these alignment fragments. This process can also be carried out automatically using a digital image capture device (e.g. a digital camera) and associated software which can identify all the separated fragments and predict their size by comparison with the in-lane markers. Preferably, for ease of identification, the in-lane markers will be tagged with another fluorophore. The same types of fluorophore that may be used to pre-stain the proteins and may be used when probing the separated proteins may also be used to enable identification of the in-lane markers.

In addition, molecular weight sizing markers can be used to assist in mapping the results of the analysis of the separated proteins and the results of the analysis of the probed proteins. The molecular weight sizing markers can be added to empty lanes on the electrophoresis gel and separated at the same time as the samples or stored digitally from previous runs.

Preferably, alignment features, molecular weight sizing markers and/or in-lane markers are used to assist in mapping the results of the analysis of the separated proteins and the results of the analysis of the probed proteins.

In embodiments where a membrane which has been treated to form isolated areas the individually separated protein transfer zones and/or the zone impermeable to liquid act as fiduciary markers to aid in alignment procedures during imaging of the membrane and the proteins contained there within.

In embodiments using more than one fluorophore (e.g. a first fluorophore for pre-staining, a second fluorophore to enable the determination of the target protein load, and a third fluorophore to enable identification of the in-lane markers) each fluorophore may excite and emit at different wavelengths such that each fluorophore can be individually identified during analysis.

The analysis of the separated proteins and/or the probed proteins may include the step of identifying the samples by reference to stored information such as a bar code which can be affixed to a gel container and/or to any holder into or onto which the gel may be transferred prior to probing. It is preferred that the barcode is resistant to chemicals used in immunodetection such as methanol. Thus a sample can be traced from when it is prepared for separation through to analysing the probed sample. Furthermore, using such means to identify and trace the sample enables additional information to be logged, e.g. the time that elapses between step b), step c), step d), and step e). The traceability and additional information provided by the means to enable identification of the extracted gel enables Good Lab Practice and Good Manufacturing Practice. Western blot techniques are also commonly used in diagnostic testing, e.g. HIV and BSE testing, where traceability throughout testing is highly beneficial.

### Detailed Description of Exemplary Embodiments

Embodiments of the present invention are illustrated in the following Examples and Figures, in which:

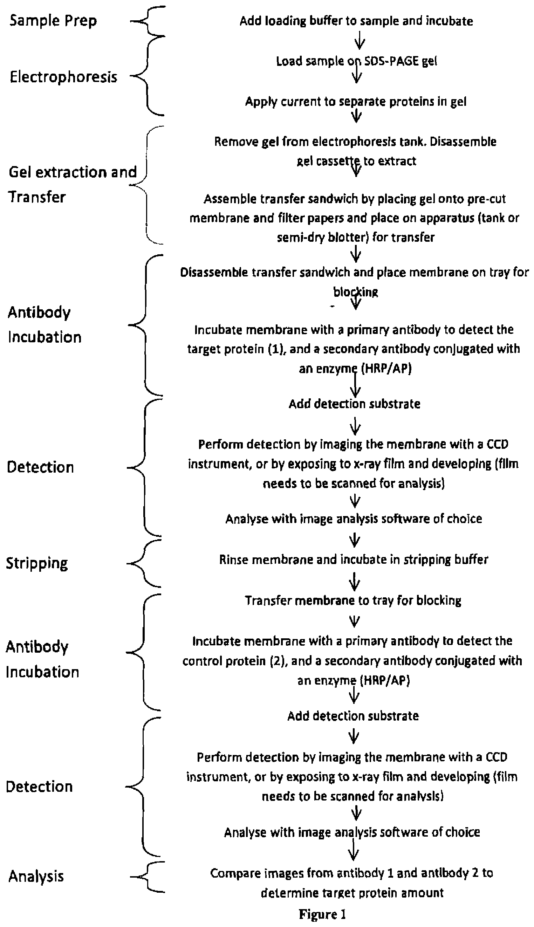

Figure 1 is a flow chart depicting one embodiment of a typical Western blot method;

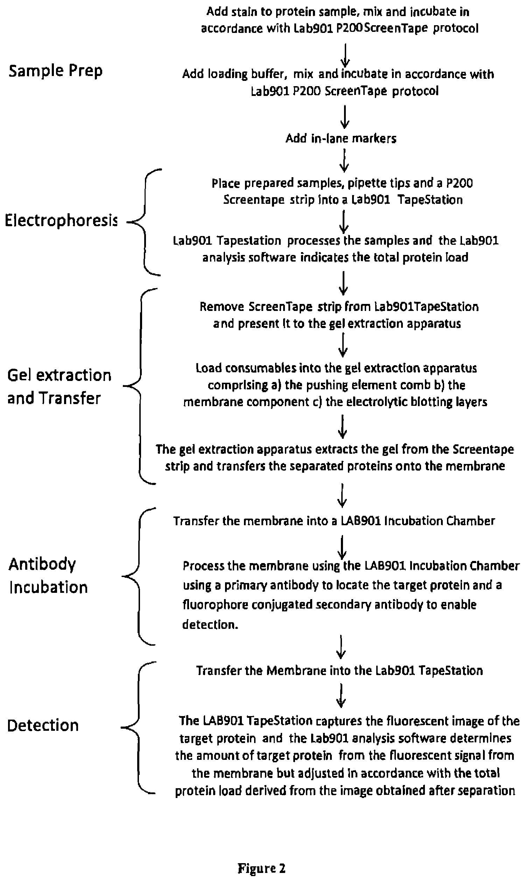

Figure 2 is a flow chart depicting one embodiment of the present invention, using Lab901's ScreenTape^{®} and TapeStation^{®} technology;
Figure 3 shows the results of analysing the separated proteins directly after separating the proteins by gel electrophoresis (NuPAGE^{®}), after the separated proteins have been transferred (Novex^{®} semi-dry transfer) and after then incubated with antibodies (SNAPid^{®}), prepared according to Example 1.

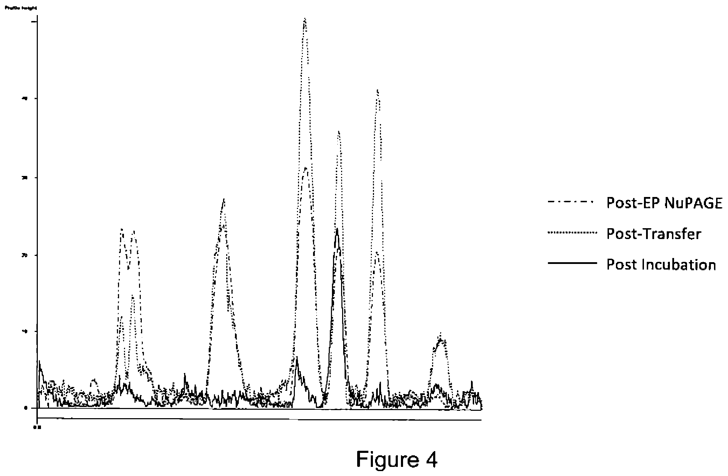

Figure 4 shows the profile comparison of total protein following electrophoresis, total protein once transferred to the membrane, and target protein prepared according to Example 1 using a PVDF membrane;
Figure 5a) shows the results of analysing the separated proteins directly after separating the proteins by gel electrophoresis using the Lab901 ScreenTape system (P200), after the separated proteins have been transferred (LAB901 ScreenTape^{®} extraction and transfer process), and after incubated with antibodies (post-Ab), prepared according to Example 2; and Figure 5b) shows the overlaid profiles obtained from Figure 5a).
Figure 6a) shows the results of analysing the separated proteins directly after separating the proteins by gel electrophoresis (P200) and after the separated proteins have been transferred (LAB901 ScreenTape^{®} extraction and transfer process), and after incubated with antibodies (post-Ab), prepared according to Example 3; and Figure 6b) shows the overlaid profiles obtained from Figure 6a).
Figure 7 shows that equivalent total protein to target protein ratios are detected independent of the dilution of total protein used in the system.
Figure 8 shows that the total protein to target protein ratio detected corresponds to the level of target protein added to the system.

### Example 1

### Gel Electrophoresis

A sample comprising proteins was prepared as follows:
a. Incubating a 20 µl protein sample with 20 µl fluorescent stain at 75 °C for 7 minutes; and
b. Adding 40 µl of a loading buffer, mixing and incubating again at 75 °C for 5 minutes.

A control sample was also prepared as follows:
a. Mixing a 20 µl protein sample with 10 µl of a reducing agent and 4 x 25 µl of Invitrogen's LDS buffer; and
b. Incubating at 75 °C for 5 minutes.

Both samples were loaded onto a NuPAGE^{®} electrophoresis gel and run according to the manufacturer's standard protocol to separate the proteins and then an image of the gel was captured using a UV transilluminator and a digital camera.

### Transferring the Separated Samples onto Membranes

The gel comprising the separated proteins was separated into two halves and then one half was transferred onto a PVDF membrane and the other half was transferred onto a nitrocellulose membrane. The separated proteins were electro-transferred onto each of the two membranes using filter papers and membranes that had been soaked in tris-glycine 20% methanol transfer buffer to assemble transfer sandwiches by placing 3 x blot paper / membrane / gel / 3 x blotting paper. Each transfer sandwich was placed in Invitrogen's Novex^{®} semi-dry transfer system and the proteins were transferred at 25V for 45 minutes.

### Probing the Separated and Transferred Samples

The separated proteins on the membranes were incubated with antibodies using an incubation apparatus (for example the Millipore's SNAPid^{®} system), as follows:
a. Placing the membranes into pre-wetted blot holder;
b. Blocking the non-specific binding sites using 0.05% non-fat dry milk (NFDM) in phosphate-buffered saline Tween (PBST);
c. Primary antibody incubation: anti-lysozyme at 1:1000 concentration and incubating for 10 minutes;
d. Washing 3 x with PBST;
e. Secondary antibody incubation: Goat anti-Rabbit IgG - HRP at 1:10,000 concentration and incubating for 10 minutes;
f. Washing 3 x with PBST;
g. Taking membranes out of blot holders and placing on cling film;
h. Adding tetramethylbenzidine (TMB) substrate and incubating for 20 minutes; and
i. Scanning the membranes.

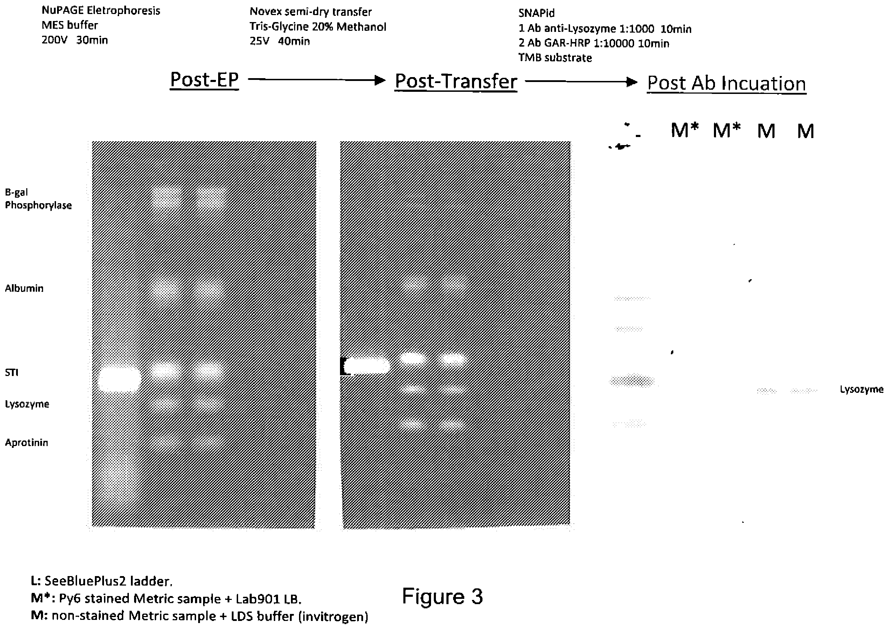

GeneTools^{®} software was used to manipulate the position of the separation such that the profiles could be overlaid for the electrophoresis medium, the membrane and the membrane post TMB. Figure 3 shows the results of the GeneTools^{®} analysis.

### Example 2

### Gel Electrophoresis

A sample comprising proteins was prepared as follows:
a. Incubating a 2 µl protein sample with 2µl fluorescent stain at 75 °C for 7 minutes;
b. Adding 4 µl of a loading buffer, mixing and incubating again at 75 °C for 5 minutes; and
c. Adding 2 µl of in-lane marker.

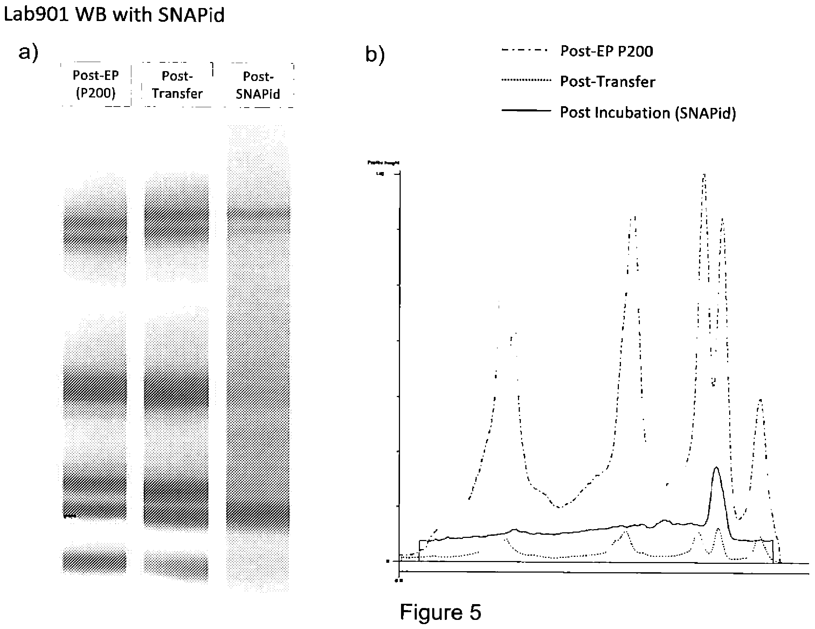

The samples were loaded onto a Lab901 P200 ScreenTape^{®} electrophoresis gel and run according to the manufacturer's standard protocol to separate the proteins. The used ScreenTape^{®} was imaged using the Lab901 TapeStation^{®} (see "P200" in Figure 5a).

### Transferring the Separated Samples onto Membranes

The used ScreenTape^{®} comprising the separated proteins was recovered from the TapeStation^{®}, its carrier layer was removed and two blades were used to cut away the top and bottom of the ScreenTape^{®} exposing the top and bottom of the gel columns contained within 16 sub-containers. A comb comprising 16 gel pushing elements was used to push against the gel within each of the sub-containers such that the gel was extracted onto a PVDF membrane that had been soaked in tris-glycine 20% methanol transfer buffer. The membrane was located on top of a sheet of blotting paper that had also been soaked in tris-glycine 20% methanol transfer buffer, with both the blotting paper and the membrane supported on an anode plate. A second sheet of blotting paper that had been soaked in tris-glycine 20% methanol transfer buffer was placed on a cathode plate and the cathode plate closed onto the anode plate, and the proteins were transferred at 50 V/cm for 10 minutes. The blotting papers and gel were removed from the membrane and the membrane was transferred to the antibody probing process.

### Quality Control Step

Post-transfer the membrane was imaged using the Lab901 TapeStation^{®}. The total protein image recorded following electrophoresis was superimposed upon the image of total protein transferred to the membrane using fiduciary markers and alignment features. The efficiency of the transfer process was then assessed before proceeding with the immunodetection process. Following this analysis the membrane was then transferred to the antibody incubation device.

### Probing the Separated and Transferred Samples

The separated proteins on the membranes were incubated with antibodies using the Millipore SNAPid^{®} system, as follows:
a. Placing the membranes into pre-wetted blot holder;
b. Blocking the non-specific binding sites using 0.05% non-fat dry milk (NFDM) in phosphate-buffered saline Tween (PBST);
c. Primary antibody incubation: anti-lysozyme at 1:1000 concentration and incubating for 10 minutes;
d. Washing 3 x with PBST;
e. Secondary antibody incubation: Goat anti-Rabbit IgG-Alexa488 at 1:10,000 concentration and incubating for 10 minutes;
f. Washing 3 x with PBST;
g. Taking membranes out of blot holders and placing on cling film;
h. Imaging the membranes on the TapeStation^{®} (see "Post Ab" in Figure 5a).

GeneTools^{®} software was used to overlay the profiles for the separated proteins and the probed proteins (Figure 5b).

### Example 3

### Gel Electrophoresis

A sample comprising proteins was prepared as follows:
a. Incubating a 2 µl protein sample with 2µl fluorescent stain at 75 °C for 7 minutes;
b. Adding 4 µl of a loading buffer, mixing and incubating again at 75 °C for 5 minutes; and
c. Adding 2 µl of in-lane marker.

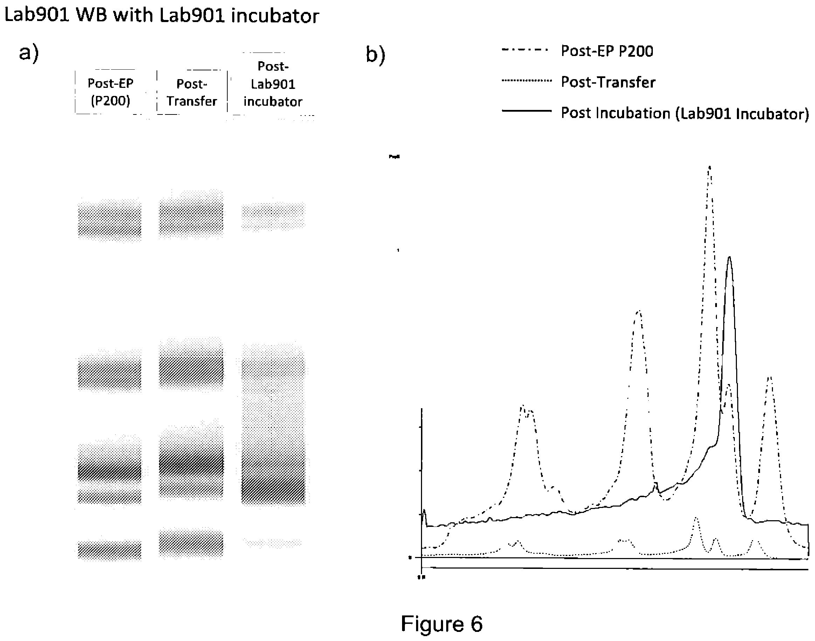

The samples were loaded onto a Lab901 P200 ScreenTape^{®} electrophoresis gel and run according to the manufacturer's standard protocol to separate the proteins. The used ScreenTape^{®} was imaged using the Lab901 TapeStation^{®} (see "P200" in Figure 6a).

### Transferring the Separated Samples onto Membranes

The used ScreenTape^{®} comprising the separated proteins was recovered from the TapeStation^{®}, its carrier layer was removed and two blades were used to cut away the top and bottom of the ScreenTape^{®} exposing the top and bottom of the gel columns contained within 16 sub-containers. A comb comprising 16 gel pushing elements was used to push against the gel within each of the sub-containers such that the gel was extracted onto a PVDF membrane that had been soaked in tris-glycine 20% methanol transfer buffer. The membrane having individual protein transfer zones created by prior heat treatment of the PVDF membrane. The membrane was located on top of a sheet of blotting paper that had also been soaked in tris-glycine 20% methanol transfer buffer, with both the blotting paper and the membrane supported on an anode plate. A second sheet of blotting paper that had been soaked in tris-glycine 20% methanol transfer buffer was placed on a cathode plate and the cathode plate closed onto the anode plate, and the proteins were transferred at a voltage of 50 V/cm for 10 minutes. The blotting papers and gel were removed from the membrane. The gel remained associated with the blotting paper post-transfer and lifting off cleanly from the membrane.

### Quality Control Step

Post-transfer the membrane was imaged using the Lab901 TapeStation^{®}. The total protein image recorded following electrophoresis was superimposed upon the image of total protein transferred to the membrane using fiduciary markers and alignment features. The efficiency of the transfer process was then assessed before proceeding with the immunodetection process. Following this analysis the membrane was then transferred to the antibody incubation device.

### Probing the Separated and Transferred Samples

The separated proteins on the membranes were transferred to an incubation apparatus , as follows:
a) The membrane composed of individual protein transfer zones surrounded by liquid impermeable sections, was relocated, either manually or automatically, onto a base plate containing sections measuring the exact dimensions of the individual protein transfer zones;
b) the membrane was positioned onto the base plate such that perfect alignment was achieved;
c) the base plate was secured atop a waste chamber containing means to connect to a vacuum manifold;
d) the membrane was then secured using an upper mask which fits over the base plate the mask containing sections measuring the exact dimensional of the individual protein transfer zones.
e) once secured into the incubation device fluid tight deformable gaskets surrounding the sections form fluid tight seals.

Immunodetection was then used to probe the membrane as follows:
a. Blocking the non-specific binding sites using 0.05% non-fat dry milk (NFDM) in phosphate-buffered saline Tween (PBST);
b. Primary antibody incubation: anti-lysozyme at 1:1000 concentration and incubating for 10 minutes;
c. Washing 3 x with PBST;
d. Secondary antibody incubation: Goat anti-Rabbit IgG-Alexa488 at 1:10,000 concentration and incubating for 10 minutes;
e. Washing 3 x with PBST;
f. Imaging the membranes on the TapeStation^{®} (see "Post Ab" in Figure 6a).

GeneTools^{®} for Lab901 software was used to overlay the profiles for the separated proteins post-electrophoresis and post-transfer and the probed proteins (Figure 6b).

### Example 4

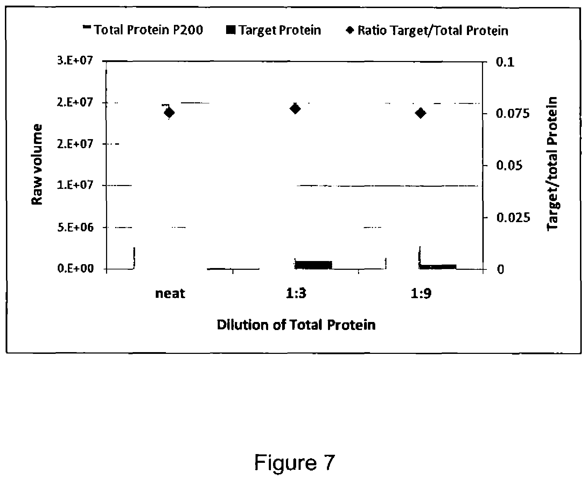

To demonstrate the sensitivity and reproducibility of the system, the target protein load to total protein load ratio was calculated for the same sample over a dilution range. An undiluted protein sample and the same sample diluted 1:3 and 1:9 were used in the LAB901 Western blot protocol (see Example 3). Total protein measurements were made in a quality control step prior to transfer of the proteins to a membrane. After the immunodetection procedure the target protein levels were measured. Comparison of the total protein load to target protein load ratios for the three concentrations, as shown in Figure 7, showed that the ratio was consistent over the dilution range. This demonstrates the sensitivity of the system as the sample with more highly concentrated proteins produced the same ratio as samples with proteins at lower concentrations. It also demonstrates the ability to use the total protein load as a loading control. Given that the same sample is loaded into different lanes or that known dilutions of a sample have been made and loaded into different lanes, any variation in total protein levels across the different lanes can be normalized to aid comparison and reproducibility. Slight variations in sample loading can often cause anomalous values during semi-quantitative Western blot analysis of proteins levels. Normalisation using the total protein loads quantified from different lanes greatly increases the reproducibility of the assay and the quality of the results.

### Example 5

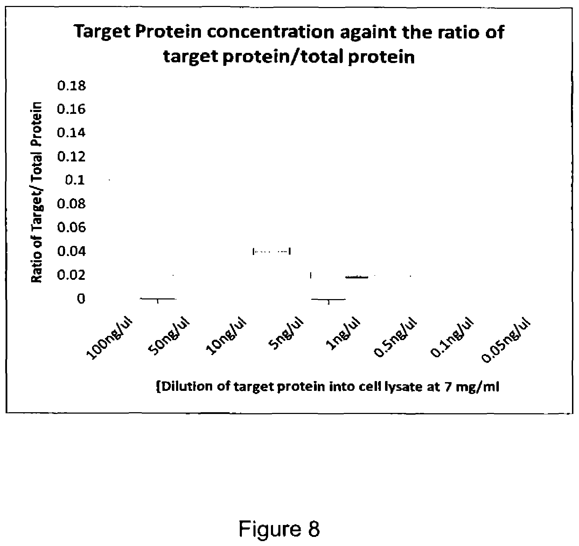

To further demonstrate the sensitivity of the system, different amounts of target protein were added to 7 mg/ml of the same cell lysate to form a dilution series. The protein samples were separated using a LAB901 ScreenTape^{®} electrophoresis gel. Following separation, the total protein load was calculated by imaging using the Lab901 TapeStation^{®} system. The proteins were then transferred to a membrane and immunodetection of the target protein performed using the LAB901 Western blot protocol (see Example 3). The total protein load was used to normalise the values of target protein load and, as can be seen in Figure 8, the ratio of target protein to total protein shoed a reduction in the target protein which corresponded to the amount of target protein added to the samples. This demonstrates the systems ability to accurately and reproducibly detect up and down regulation of proteins.

## Claims

1. A method of performing the Western blot analytical technique, wherein the method is carried out in the following order:
a). pre-staining the proteins within a sample;
b). separating the proteins using gel electrophoresis;
c). analysing the separated proteins to determine the total protein load and/or at least one housekeeping protein load;
d). transferring the proteins onto at least one membrane;
e). probing the separated proteins to detect a target protein; and
f). analysing the probed proteins to determine the target protein's molecular weight and load,
wherein the total protein load and/or housekeeping protein load are compared to the target protein load to find the ratio of total protein and/or housekeeping protein load to target protein load, and wherein the total protein load and/or housekeeping protein load are used as a loading control and the ratio of the total protein and/or housekeeping protein load to target protein load is normalised using these values.

2. A method according to claim 1, wherein the total protein load is determined to be the complete area under the curve of a plot of fluorescence intensity against position of said fluorescence in an electrophoresis gel or plotted against the time said fluorescence is detected at a fixed point from the start of protein separation
and/or wherein the target protein load and/or housekeeping protein load is determined to be the area under a specific peak on a plot of fluorescence intensity against position of said fluorescence in an electrophoresis gel or plotted against the time said fluorescence is detected at a fixed point from the start of protein separation.

3. A method according to claim 1 or 2,
wherein the concentration of target protein is calculated
(i) using the load of known concentrations of a protein to create a calibration curve and plotting the target protein load on said calibration curve or
(ii) using the load of known concentrations of in lane markers and comparing these values to the target protein load
and/or wherein a serial dilution of the target protein is performed to increase accuracy of the calculation to determine the target protein concentration.

4. A method according to any preceding claim, wherein the proteins within the sample are pre-stained using at least one fluorophore,
in particular wherein more than one fluorophore is used, and each fluorophore excites and emits at different wavelengths such that each fluorophore can be individually recognised during analysis,
in particular wherein the total protein and/or house keeping protein are associated with a fluorophore and the target protein is associated with fluorophore which excites and emits at a different wavelength from the fluorophore associated with the total protein and/or housekeeping protein.

5. A method according to any preceding claim,
wherein analysing the separated proteins to determine the total protein load comprises capturing an image of the separated proteins and/or
wherein analysing the probed proteins to determine the target protein load comprises capturing an image of the probed proteins.

6. A method according to any preceding claim, wherein
(i) alignment features are used, and/or
(ii) in-lane markers are added during step b)., and/or
(iii) molecular weight sizing markers are used
to assist in mapping the results of the analysis of the separated proteins and the results of the analysis of the probed proteins.

7. A method according to any of the preceding claims, wherein the method comprises capturing an image of the electrophoresis gel post transfer as a quality control step for the efficiency of protein transfer
and/or wherein the method comprises capturing an image of the separated proteins after they have been transferred onto the at least one membrane.

8. A method according to any preceding claim, wherein a molecular separation gel is extracted from a container by moving the gel relative to the container.

9. A method according to claim 8, wherein the means to extract the molecular separation gel from the container is adapted to move the gel relative to the container
(i) whilst maintaining the aspect ratio of the gel or
(ii) by pushing the gel.

10. A method according to claim 9, wherein the means to extract the molecular separation gel from the container by pushing the gel comprises a plurality of gel pushing elements acting on a plurality of gel sub-containers.

11. A method according to claim 10,
wherein the gel pushing elements form a comb
and/or
wherein the gel pushing elements are guided into location.

12. A method according to any of the proceeding claims, wherein the immunodetection incubation is carried out in an incubation device adapted to incubate sections of at least one membrane individually.

13. A method according to claim 12, wherein the incubation device comprises a mask and at least one membrane, wherein apertures in the mask form isolated sections, in particular wherein the isolated sections are formed by the mask comprising raised barriers, and wherein at least one membranes is located within each of the isolated sections.

14. A method according to any preceding claim, wherein the least one membrane is separated into individual protein transfer zones surrounded by the zone impermeable to liquid.

15. A method according to claim 14, wherein individual protein transfer zones are created by heat treatment or by ultrasonic welding.

## Patentansprüche

1. Ein Verfahren zum Durchführen der Western Blot Analysetechnik, wobei das Verfahren in der folgenden Reihenfolge ausgeführt wird:
a). Vorfärben der Proteine in einer Probe;
b). Trennen der Proteine unter Verwendung einer Gelelektrophorese;
c). Analysieren der getrennten Proteine zum Bestimmen der gesamten Proteinladung und/oder zumindest einer Haushaltsproteinladung;
d). Transferieren der Proteine auf zumindest eine Membran;
e). Untersuchen der getrennten Proteine zum Detektieren eines Zielproteins; und
f). Analysieren der untersuchten Proteine zum Bestimmen des Molekulargewichts und der Ladung des Zielproteins,
wobei die gesamte Proteinladung und/oder die Haushaltsproteinladung mit der Zielproteinladung verglichen werden, um das Verhältnis von der gesamten Proteinladung und/oder der Haushaltsproteinladung zu der Zielproteinladung zu finden, und
wobei die gesamte Proteinladung und/oder die Haushaltsproteinladung als eine Ladekontrolle verwendet werden und das Verhältnis der gesamten Proteinladung und/oder der Haushaltsproteinladung zu der Zielproteinladung unter Verwendung dieser Werte normalisiert wird.

2. Ein Verfahren gemäß Anspruch 1, wobei bestimmt wird, dass die gesamte Proteinladung die vollständige Fläche unter der Kurve eines Plots der Fluoreszenzintensität gegen eine Position der Fluoreszenz in einem Elektrophoresegel oder geplottet gegen die Zeit, während welcher die Fluoreszenz an einem festen Punkt detektiert wird, von dem Beginn der Proteintrennung an, ist
und/oder wobei bestimmt wird, dass die Zielproteinladung und/oder die Haushaltsproteinladung die Fläche unter einem spezifischen Peak auf einem Plot der Fluoreszenzintensität gegen die Position der Fluoreszenz in einem Elektrophoresegel oder geplottet gegen die Zeit, während welcher die Fluoreszenz an einem festen Punkt detektiert wird, von dem Beginn der Proteintrennung an, ist.

3. Ein Verfahren gemäß Anspruch 1 oder 2,
wobei die Konzentration des Zielproteins berechnet wird
(i) unter Verwendung der Ladung von bekannten Konzentrationen eines Proteins zum Erzeugen einer Kalibrationskurve und mittels Plottens der Zielproteinladung auf die Kalibrationskurve oder
(ii) unter Verwendung der Ladung von bekannten Konzentrationen von in-Bahn Markern und mittels Vergleichens dieser Werte mit der Zielproteinladung
und/oder wobei eine serielle Verdünnung des Zielproteins zum Erhöhen der Genauigkeit der Berechnung zum Bestimmen der Zielprotein Konzentration durchgeführt wird.

4. Ein Verfahren gemäß irgendeinem vorangehenden Anspruch, wobei die Proteine in der Probe vorgefärbt werden unter Verwendung von zumindest einem Fluorophor,
insbesondere wobei mehr als ein Fluorophor verwendet wird und jedes Fluorophor bei verschiedenen Wellenlängen anregt und emittiert, so dass jedes Fluorophor während der Analyse individuell erkannt werden kann,
insbesondere wobei das gesamte Protein und/oder das Haushaltsprotein mit einem Fluorophor assoziiert sind und das Zielprotein mit einem Fluorophor assoziiert ist, welches bei einer unterschiedlichen Wellenlängen anregt und emittiert als das Fluorophor, welches mit dem gesamten Protein und/oder dem Haushaltsprotein assoziiert ist.

5. Ein Verfahren gemäß irgendeinem vorangehenden Anspruch,
wobei das Analysieren der getrennten Proteine zum Bestimmen der gesamten Proteinladung aufweist
Erfassen eines Bildes des getrennten Proteins und/oder
wobei das Analysieren der untersuchten Proteine zum Bestimmen der Zielproteinladung aufweist
Erfassen eines Bildes der untersuchten Proteine.

6. Ein Verfahren gemäß irgendeinem vorangehenden Anspruch, wobei
(i) Ausrichtungsmerkmale verwendet werden, und/oder
(ii) in-Bahn Marker während Schritt b). hinzugefügt werden, und/oder
(iii) Molekulargewicht Größenmarker verwendet werden zum Unterstützen des Abbildens der Ergebnisse der Analyse der getrennten Proteine und der Ergebnisse der Analyse der untersuchten Proteine.

7. Ein Verfahren gemäß irgendeinem der vorangehenden Ansprüche, wobei das Verfahren aufweist
Erfassen eines Bildes des Elektrophoresegels nach dem Transfer als ein Qualitätskontrollschritt für die Effizienz des Protein Transfers
und/oder wobei das Verfahren aufweist
Erfassen eines Bildes der getrennten Proteine nachdem sie auf die zumindest eine Membran transferiert wurden.

8. Ein Verfahren gemäß irgendeinem vorangehenden Anspruch, wobei ein molekulares Trenrigel von einem Container mittels Bewegens des Gels relativ zu dem Container extrahiert wird.

9. Ein Verfahren gemäß Anspruch 8, wobei das Mittel zum Extrahieren des molekularen Trenngels von dem Container eingerichtet ist zum Bewegen des Gels relativ zu dem Container
(i) während das Aspektverhältnis des Gels aufrechterhalten wird oder
(ii) mittels Drückens des Gels.

10. Ein Verfahren gemäß Anspruch 9, wobei das Mittel zum Extrahieren des molekularen Trenngels von dem Container mittels Drückens des Gels aufweist
eine Mehrzahl von Gel Drückelementen, welche auf eine Mehrzahl von Gel Teil-Containern wirken.

11. Ein Verfahren gemäß Anspruch 10,
wobei die Gel Drückelemente eine Wabe bilden
und/oder
wobei die Gel Drückelemente in eine Position geführt werden.

12. Ein Verfahren gemäß irgendeinem der vorangehenden Ansprüche, wobei die Immundetektion Inkubation in einer Inkubationsvorrichtung ausgeführt wird, welche eingerichtet ist zum individuellen Inkubieren von Abschnitten der zumindest einen. Membran.

13. Ein Verfahren gemäß Anspruch 12, wobei die Inkubationsvorrichtung aufweist
eine Maske und zumindest eine Membran, wobei Öffnungen in der Maske isolierte Abschnitte bilden, insbesondere wobei die isolierten Abschnitte mittels der Maske gebildet sind, welche angehobenen Barrieren aufweist, und wobei zumindest eine Membran in jedem der isolierten Abschnitte angeordnet ist.

14. Ein Verfahren gemäß irgendeinem vorangehenden Anspruch, wobei die zumindest eine Membran in einzelne Protein Transferzonen unterteilt ist, welche von der Zone umgeben sind, welche undurchlässig für Flüssigkeit ist.

15. Ein Verfahren gemäß Anspruch 14, wobei die einzelnen Protein Transferzonen mittels Wärmebehandelns oder mittels Ultraschallschweißens erzeugt werden.

## Revendications

1. Procédé de réalisation de la technique analytique de transfert de Western, dans lequel le procédé est réalisé dans l'ordre suivant :
a) la coloration préalable des protéines au sein d'un échantillon ;
b) la séparation des protéines par électrophorèse sur gel ;
c) l'analyse des protéines séparées pour déterminer le chargement en protéine totale et/ou au moins un chargement en protéine domestique ;
d) le transfert des protéines sur au moins une membrane ;
e) le sondage des protéines séparées pour détecter une protéine cible ; et
f) l'analyse des protéines sondées pour déterminer le poids moléculaire de la protéine cible et le chargement en celle-ci,
dans lequel le chargement en protéine totale et/ou le chargement en protéine domestique sont comparés au chargement en protéine cible pour déterminer le rapport entre le chargement en protéine totale et/ou en protéine domestique et le chargement en protéine cible, et dans lequel le chargement en protéine totale et/ou le chargement en protéine domestique sont utilisés comme témoin de chargement et le rapport entre le chargement en protéine totale et/ou en protéine domestique et le chargement en protéine cible est normalisé en utilisant ces valeurs.

2. Procédé selon la revendication 1, dans lequel le chargement en protéine totale est déterminé pour être la surface complète sous la courbe d'un tracé de l'intensité de fluorescence par rapport à la position de ladite fluorescence dans un gel d'électrophorèse ou tracée par rapport au temps que ladite fluorescence soit détectée à un point fixe à partir du début de la séparation des protéines
et/ou dans lequel le chargement en protéine cible et/ou le chargement en protéine domestique sont déterminés pour être la surface sous un pic spécifique sur un tracé de l'intensité de fluorescence par rapport à la position de ladite fluorescence dans un gel d'électrophorèse ou tracée par rapport au temps que ladite fluorescence soit détectée à un point fixe à partir du début de la séparation des protéines.

3. Procédé selon la revendication 1 ou 2,
dans lequel la concentration eh protéine cible est calculée
(i) en utilisant le chargement de concentrations connues d'une protéine pour créer une courbe d'étalonnage et en traçant le chargement en protéine cible sur ladite courbe d'étalonnage ou
(ii) en utilisant le chargement de concentrations connues de marqueurs en couloir et en comparant ces valeurs au chargement en protéine cible
et/ou dans lequel une dilution en série de la protéine cible est réalisée pour augmenter la précision du calcul pour déterminer la concentration en protéine cible.

4. Procédé selon l'une quelconque des revendications précédentes, dans lequel les protéines au sein de l'échantillon sont préalablement colorées en utilisant au moins un fluorophore,
en particulier dans lequel plus d'un fluorophore est utilisé, et chaque fluorophore s'excite et émet à des longueurs d'onde différentes de façon que chaque fluorophore puisse être reconnu individuellement lors d'une analyse,
en particulier dans lequel la protéine totale et/ou la protéine domestique sont associées à un fluorophore et la protéine cible est associée à un fluorophore qui s'excite et émet à une longueur d'onde différente de celle du fluorophore associé à la protéine totale et/ou la protéine domestique.

5. Procédé selon l'une quelconque des revendications précédentes,
dans lequel l'analyse des protéines séparées pour déterminer le chargement en protéine totale comprend la capture d'une image des protéines séparées et/ou
dans lequel l'analyse des protéines sondées pour déterminer le chargement en protéine cible comprend la capture d'une image des protéines sondées.

6. Procédé selon l'une quelconque des revendications précédentes, dans lequel
(i) des caractéristiques d'alignement sont utilisées, et/ou
(ii) des marqueurs en couloir sont ajoutés lors de l'étape b), et/ou
(iii) des marqueurs de dimensionnement de poids moléculaire sont utilisés
pour faciliter le mappage des résultats de l'analyse des protéines séparées et des résultats de l'analyse des protéines sondées.

7. Procédé selon l'une quelconque des revendications précédentes, dans lequel le procédé comprend la capture d'une image du gel d'électrophorèse après transfert en tant qu'étape de contrôle qualité pour l'efficacité du transfert des protéines
et/ou dans lequel le procédé comprend la capture d'une image des protéines séparées après leur transfert sur l'au moins une membrane.

8. Procédé selon l'une quelconque des revendications précédentes, dans lequel un gel de séparation moléculaire est extrait à partir d'un récipient en déplaçant le gel par rapport au récipient.

9. Procédé selon la revendication 8, dans lequel le moyen pour extraire le gel de séparation moléculaire à partir du récipient est adapté pour déplacer le gel par rapport au récipient
(i) tout en conservant le rapport d'aspect du gel ou
(ii) en poussant le gel.

10. Procédé selon la revendication 9, dans lequel le moyen pour extraire le gel de séparation moléculaire à partir du récipient en poussant le gel comprend une pluralité d'éléments poussant le gel agissant sur une pluralité de sous-récipients de gel.

11. Procédé selon la revendication 10,
dans lequel les éléments poussant le gel forment un peigne
et/ou
dans lequel les éléments poussant le gel sont guidés dans un emplacement.

12. Procédé selon l'une quelconque des revendications précédentes, dans lequel l'incubation d'immunodétection est réalisée dans un dispositif d'incubation adapté pour mettre en incubation individuellement des sections d'au moins une membrane.

13. Procédé selon la revendication 12, dans lequel le dispositif d'incubation comprend un masque et au moins une membrane, dans lequel des ouvertures dans le masque forment des sections isolées, en particulier dans lequel les sections isolées sont formées par le masque comprenant des barrières élevées, et dans lequel au moins une membrane est située au sein de chacune des sections isolées.

14. Procédé selon l'une quelconque des revendications précédentes, dans lequel l'au moins une membrane est séparée en zones individuelles de transfert de protéine entourées par la zone imperméable au liquide.

15. Procédé selon la revendication 14, dans lequel les zones individuelles de transfert de protéine sont créées par traitement thermique ou par soudage aux ultrasons.
